# EUROPEAN PATENT SPECIFICATION

(11) **EP 1 198 239 B1**
(45) Date of publication and mention of the grant of the patent: **26.09.2007**
(21) Application number: 00946124.5
(22) Date of filing: 17.07.2000
(51) Int. Cl.: A61K 38/16, A61K 39/02, A61K 48/00, A61K 45/06, A61K 31/713, A61P 35/00

(54) **METHODS EMPLOYING BACTERIAL ParD kis/ParD kid TOXIN-ANTITOXIN SYSTEM FOR KILLING EUKARYOTIC CELLS**
VERWENDUNG VON BAKTERIELLEN ParD kis/ParD kid TOXIN-ANTITOXIN SYSTEM ZUR TÖTUNG VON EUKARYOTISCHEN ZELLEN
METHODES EMPLOYANT LE SYSTEME TOXINE-ANTITOXINE BACTERIEN ParD kis/ParD kid POUR TUER DES CELLULES EUCARYOTES

(30) Priority: 16.07.1999 GB 9916810
(43) Date of publication of application: 24.04.2002
(73) Proprietor: Cancer Research Ventures Limited, London NW1 4JL (GB)
(72) Inventor: DE LA CUEVA MENDEZ, Guillermo, 28049 Madrid (ES); LASKEY, Ronald Alfred, Cambridge CB2 1QR (GB); MILLS, Anthony David, Cambridge CB2 1QR (GB); DIAZ OREJAS, Ramon, 28006 Madrid (ES)
(74) Representative: Walton, Seán Malcolm
(86) International application number: PCT/GB2000/002743
(87) International publication number: WO 2001/005421

(56) References cited:
- WO-A-94/26308
- WO-A-99/58652
- HAMBLETON ET AL.: "Antitoxins and botulinum toxin treatment" BRITISH MEDICAL JOURNAL, vol. 304, 1992, pages 959-960, XP000944661
- M. HOLCIK ET AL.: "Conditionally lethal genes associated with bacterial plasmids." MICROBIOLOGY, vol. 143, 1997, pages 3403-3416, XP000941408
- MAGNUSON R. ET AL.: "Corepression of the P1 operon by Phd and Doc." J. BACTERIOL., vol. 180, no. 23, 1998, pages 6342-6351, XP000942967
- RAWLINGS D.E.: "Proteic toxin-antitoxin, bacterial plasmid addiction systems and their evolution with special reference to the ps system of pTF-FC2." FEMS MICROBIOL. LETTERS, vol. 176, 15 July 1999 (1999-07-15), pages 269-277, XP000942964

## Description

The present invention relates to killing cells, or at least impeding cell cycle progression. More particularly it relates to methods and means for attacking eukaryotic cells, such as tumour cells, with cytostatic, cytotoxic and/or cytopathic agents. Specifically, the present invention employs the ParD kid toxin and ParD kis antitoxin under appropriate control for selective cell cycle inhibition and/or killing of target cells.

There are various contexts in which it is desired to kill cells, in particular selectively to kill certain cells within a population of cells. In some contexts inhibition of cellular growth or proliferation, for instance by impeding cell cycle progression, may be sufficient. For simplicity herein, unless context provides otherwise, reference to killing cells may be used to encompass such inhibition.

An important area of application is in treatment of tumours, cancer, psoriasis, arteriosclerosis and other hyper-proliferative disorders. Other applications of embodiments of the present invention include targeting any desired eukaryotic cell for killing or at least inhibition of growth. This may include cell lineage knock-outs and targeted cell ablation, for instance in developmental control, or organogenesis studies. *In vitro* applications include study of the control or replication in prokaryotic and/or eukaryotic cells, screening for an antidote for a toxin, or toxin inhibited by an antidote, design of or screening for improved toxin and/or antidote factors, and analysis of physiological responses of different cell types to inhibition of cell progression and/or inhibition of DNA replication.

In plants, pathogen defence responses involve cell necrosis, for instance triggered at a site of pathogen infection or ingress. Induced resistance is strongly correlated with the hypersensitive response (HR), an induced response associated with localized cell death at sites of attempted pathogen ingress. It is hypothesized that by HR the plant deprives the pathogen of living host cells.

Many plant defence mechanisms are strongly induced in response to a challenge by an unsuccessful pathogen. Such an induction of enhanced resistance can be systemic. It is believed that when a plant is challenged by a pathogen to which it is resistant, it undergoes an HR at the site of attempted ingress of the incompatible pathogen. The induced HR leads to a systemic enhancement and acquisition of plant resistance to virulent pathogens that would normally cause disease in the unchallenged plant.

Artificial induction of cell death in plants has been shown to be able to provide pathogen resistance, even where the mechanism inducing cell death is not triggered by any pathogen resistance gene. For instance, genes coding for substances leading to rapid cell death, such as BARNASE or diphtheria toxin may be use to induce the changes that lead to acquired resistance even though cell death in these latter examples is not caused by activation of the defence response. BARNASE is a ribonuclease from *Bacillus amyloliquifaciens* (Hartley (1988) J. Mol. Biol. 202: 913-915; Hartley (1989) Trends Biochem. Sci. 14: 450-454) and there is a corresponding protein called BARSTAR which inhibits BARNASE by forming a complex with it.

Use of embodiments of the present invention in plants may be used to generate protection against attack from fungi, bacteria, viruses or nematodes.

Plants of particular interest for use in embodiments of the present include cereals, maize, corn, wheat, barley, oats, rice, Brassicas, curcubits, potatoes, tomatoes, cotton, soya bean, and carrot.

Another use of embodiments of the present invention in plants include generation of male sterility(Mariani et al. Nature 357 384-387). For instance toxin or a toxin system in accordance with the present invention may be introduced into plants under appropriate control for tapetal-specific expression (Seurinck et al. (1990) Nucleic Acids Res. 18: 3403; Koltunow et al. (1990) Plant Cell 2, 1201-1224; Mariani et al (1990) Nature 347: 737-741). Male sterility in plants facilitates hybrid seed generation by preventing self-pollination, allowing agriculturalists to take advantage of so-called "hybrid vigour" by which crosses between inbred plant lines often result in progeny with higher yield and increased resistance to disease. Provision of horticultural or ornamental plants lacking ability to make pollen may be used to reduce allergy problems of local inhabitants or for aesthetic reasons (e.g. in lilies, where anthers are currently removed by hand).

A further use in plants is in generation of seedlessness, often desirable for convenience and taste in produce such as watermelons, grapes, oranges and related fruits, tomatoes, peppers, cucumbers and so on. Toxin can be placed under regulatory control of a seed-specific promoter, such as the promoter of a seed storage protein (Higgins et al, (1984) Ann. Rev. Plant. Physiol. 35: 191-221; Goldberg et al (1989) Cell 56: 149-160). Examples of seed-specific promoters include those for bean β-phaseolin (Sengupta-Gopalan et al, (1985) PNAS US 82: 3320-3324), bean lectin (Voelker et al (1987) EMBO J. 6: 3571-3577), soybean lectin (Ocamuro et al. (1986) PNAS USA 83: 8240-8344), rapeseed napin (Radke et al. Theor. Appl. Genet. 75: 685-694), maize zein (Hoffman et al (1987) EMBO J. 6: 3213-3221), barley β-hordein (Marris et al (1988) Plant Mol. Biol. 10: 359-366) and wheat glutenin (Colot et al. (1987) EMBO J. 6: 3559-3564).

Prokaryotic plasmids have developed different genetic systems that increase their stable maintenance in bacterial hosts. These systems are classified into two different types: partition systems, that ensure a well controlled partition of plasmid DNA copies between the two daughter cells, and killer systems, that eliminate from the bacterial population those daughter cells that have lost the plasmid during division (Yarmolinsky, Science (1995) Feb 10,267(5199): 836-7). The latter are composed of two components: a bacterial toxin (always a protein), and its antidote (a protein or an antisense RNA that inhibits transcription of its killer partner) (Jensen and Gerdes, Mol. Microbiol. (1995) Jul. 17 (2): 205-10; Thisted et al. J. Mol. Biol. (1992). Jan. 5 223 (1): 41-54). These killer systems are generally organized similarly from a molecular point of view, and several mechanisms ensure that a typical killer system is not activated if the stability of its harbouring plasmid is not compromised. Thus, both proteic antidote and toxic components are organized in a bicistronic operon, and the system is molecularly designed in such a way that both transcriptional and translational processes are optimised to maintain it in a silent state (i.e. a state in which the toxic component is being neutralised by its antidote) (Jensen and Gerdes, Mol. Microbiol. (1995) Jul 17(2): 205-10; Holcik and Iyer, Microbiology (1997), 143: 3403-3416).

Under normal circumstances, both components of a killer system are synthesized at a basal level in the host by its harbouring plasmid, allowing the host to survive. If a segregant bacteria (i.e. a bacteria that has lost the plasmid) appears after cell division, another characteristic of these systems allows activation of the killing process in order to counter-select that specific cell: that is, the stability of the antidote is lower than the toxin. Thus, without a continuous synthesis of the antidote, its preferential degradation leads to the appearance of a non-neutralised toxin that is then able to exert its lethal effect over the host. This toxic effect can be executed affecting different cellular targets, depending on the specific killer system, for example DnaB dependent replication (parD, pem), DNA-gyrase complex (ccd), protein synthesis inhibition (KicB), and septum formation (kil), (for references see Holcik and Iyer, Microbiology (1997), 143, 3403-3416). Yarmolinsky describes in Science, Vol. 267 (1995) other putative "addiction molecules" like the type II restriction enzymes (putative toxins) Pae R7 and EcoRI and their cognate methylases, that enhance the apparent stability of their harbouring plasmids (the original reference for this addiction modules is in Naito et al. Science 267:897 (1995)). In this work, Yarmolinsky also describes a couple of putative killer systems from bacteriophage lambda (Rex protein) and a couple of strains of *E. coli* carrying the gene cluster prr, that encodes for an anticodon nuclease that can be activated by a 26 residue polypeptide from bacteriophage T4 and can then cleave a transfer RNA important for lysine incorporation into proteins. T4 is invulnerable to this protein because it encodes for a couple of otherwise non-essential proteins that undoes the damage. He also describes strains of *E. coli* that carry defective prophage e14, and that accomplish exclusion by cleavage of elongation factor Tu and inhibiting translation globally.

ParD is one of these killer systems (Bravo et al. Mol. Gen. Genet. (1987) Nov. 210(1): 101-10; Bravo et al. Mol. Gen. Genet. (1988). Dec. 215(1): 146-51). It is encoded by Gram negative plasmid R1 and is composed of two genes: kis (for killing suppressor) and kid (for killing determinant) that encode for the antidote (10 KDa) and the toxin (12 KDa) respectively. ParD is a cryptic killer system that is tightly regulated to avoid its activation under circumstances that do not compromise R1 stability. Thus, it is controlled by coupled transcription (Ruiz-Echevarria et al. Mol. Microbiol. (1991) Nov. 5(11): 2685-93), by post-transcriptional processing of its bicistronic mRNA (Ruiz-Echevarria et al. Mol. Gen. Genet. (1995) Sep. 20 248(5): 599-609), by overlapped translation (Ruiz-Echevarria et al. Mol. Gen. Genet. (1995) Sep. 20 248(5): 599-609), and by a very tight interaction between Kis and Kid to form a non-toxic complex that, at the same time, is able to repress transcription from its own promoter (Ruiz-Echevarria et al. Mol. Microbiol. (1991) Nov. 5(11): 2685-93). Genetic organisation of *ParD* favours coupled transcription, overlapped translation and post-transcriptional modification of some of the obtained mRNA. Kis/Kid complexes repress transcription of *kis* and *kid* genes.

ParD homologues have been described at least in plasmid R100 (pem system) (Tsuchimoto et al. J. Bacteriol. (1988) Apr. 170(4): 1461-6; Tsuchimoto et al. J. Bacteriol. (1992) Jul. 174(13): 4205-11; Tsuchimoto et al. Mol. Gen. Genet. (1993) Feb. 237(1-2): 81-88); Masuda et al. J. Bacteriol. (1993) Nov. 175(21): 6850-6) and in *E. coli* chromosome (ChpA and ChpB systems) (Tsuchimoto et al. Mol. Gen. Genet. (1993) Feb. 237(1-2): 81-88). Others are revealed by database searching.

Kid inhibits initiation of replication of the E. *coli* genome and of DnaB (i.e. the main replicative helicase of *E. coli)* dependent replication plasmids (Ruiz-Echevarria et al. J. Mol. Biol. (1995) Apr. 7 247(4): 568-77), and over-expression of the latter titrates the toxic effect of the former in this organism *in vivo* (Ruiz-Echevarria et al. J. Mol. Biol. (1995) Apr. 7 247(4): 568-77), suggesting that DnaB is involved in the mechanism of inhibition by Kid. Recent observations in the inventors' laboratory strongly suggest that this inhibition is due neither to disassembly by Kid of DnaB hexameric complexes in solution nor to inhibition of its helicase activity over a wide range of substrates including oriC, the replication origin of the *E. coli* genome. Without wishing to be limited by theory, it may be that loading of DnaB at the origin of replication is the process inhibited by Kid, either by direct interaction between them and/or mediated by a third component (DNA or protein) yet to be described. Current research is focused on the identification of the exact mechanism of action of Kid from a molecular point of view.

Until the work of the present inventors disclosed herein it was not obvious that prokaryotic systems that have evolved for specific roles in bacteria could function in eukaryotic cells.

For instance, in a two-component killer system such as involving *kis*/*kid,* both components need to perform their respective functions - the toxin to kill cells in the absence of antidote (or when present in excess of antidote), and the antidote to.both neutralise the toxin and be controllable, for instance by a mechanism involving rapid turnover. Preferably the toxin does not exert any side effect on cell viability. Rather, it is preferred that cell killing is via a programmed cell death mechanism such as apoptosis. In plants it may be preferred for certain applications to induce a necrotic response, e.g. in inducing or enhancing pathogen resistance.

The present inventors have shown that bacterial toxin and antidote are functional in eukaryotic cells, yeast, *Xenopus* and mammalian (in particular human), and can be controlled to inhibit cell cycle progression and cellular proliferation and to kill cells. It is shown in experiments described below that cells can be killed by apoptosis.

### Brief Description of the Figures

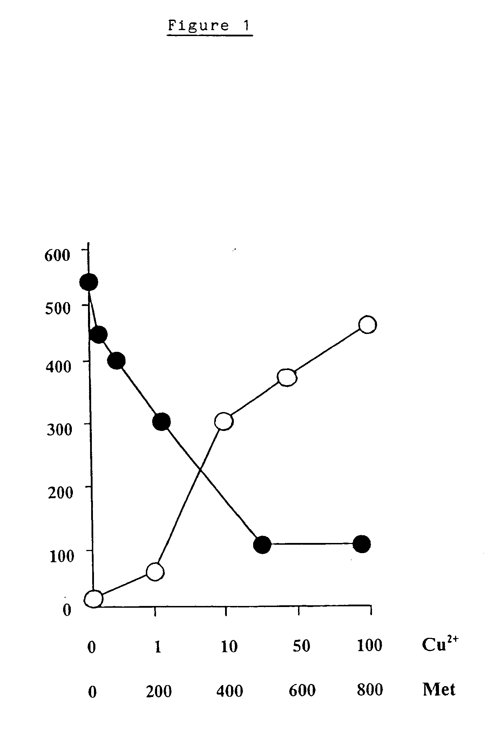

Figure 1 shows results of experiments showing that a promoter induced by Curl later used for control of Kis antidote expression and a different promoter repressed by methionine later used for control of Kid toxin expression are both functional in *S. cerevisiae.* The graph shows units of β-galactosidase activity at different concentrations (µM) of the regulatory factors Cu²⁺ (light circles) and Met (dark circles).

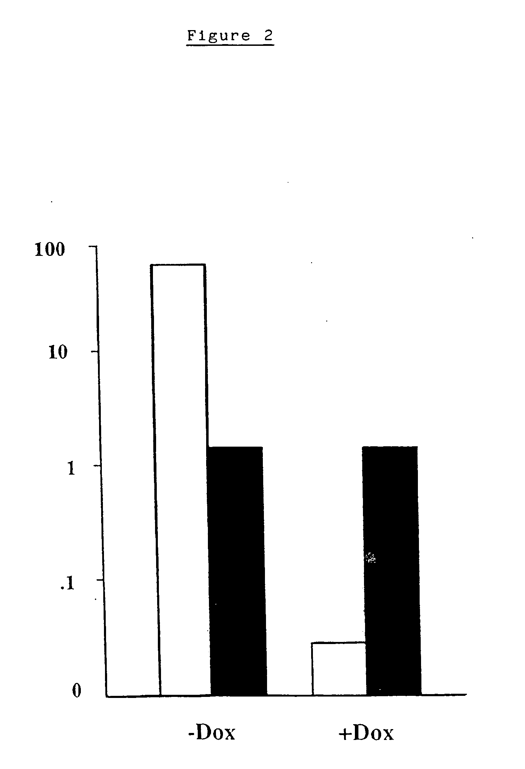

Figure 2 shows results of experiments demonstrating the effect of Doxycyclin on a Tetracyclin regulatable promoter (Tet Pr) activity in HeLa cells (light bars), this promoter later used for control of expression of antidote Kis (in the vector pTRE-Luc), and an absence of effect of Doxycyclin on Cytomegalovirus Early promoter (CMV Pr) activity (dark bars), this promoter later used for control of expression of toxin Kid (pCMV-Luc). Luciferase activity is plotted, in arbitrary units.

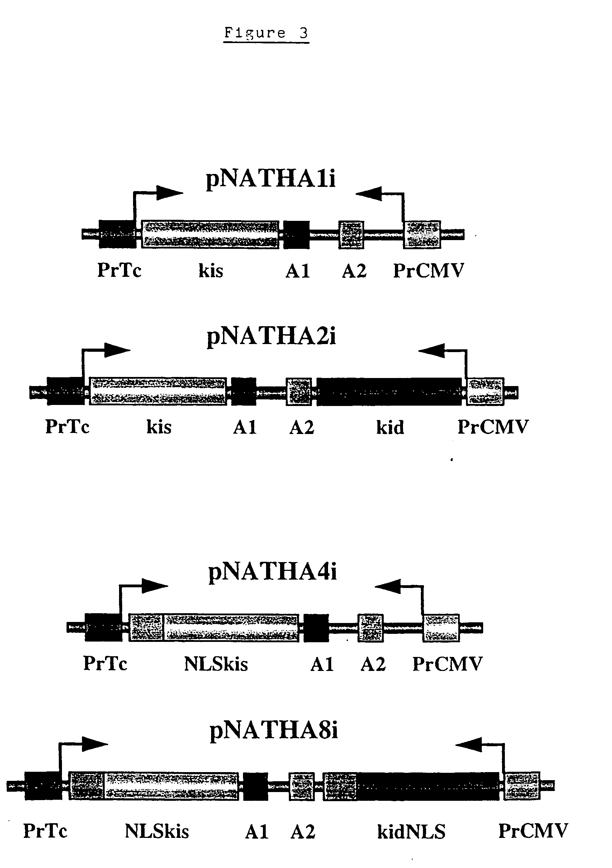

Figure 3 illustrates various constructs employed for expression of Kis and/or Kid in HeLa cells.

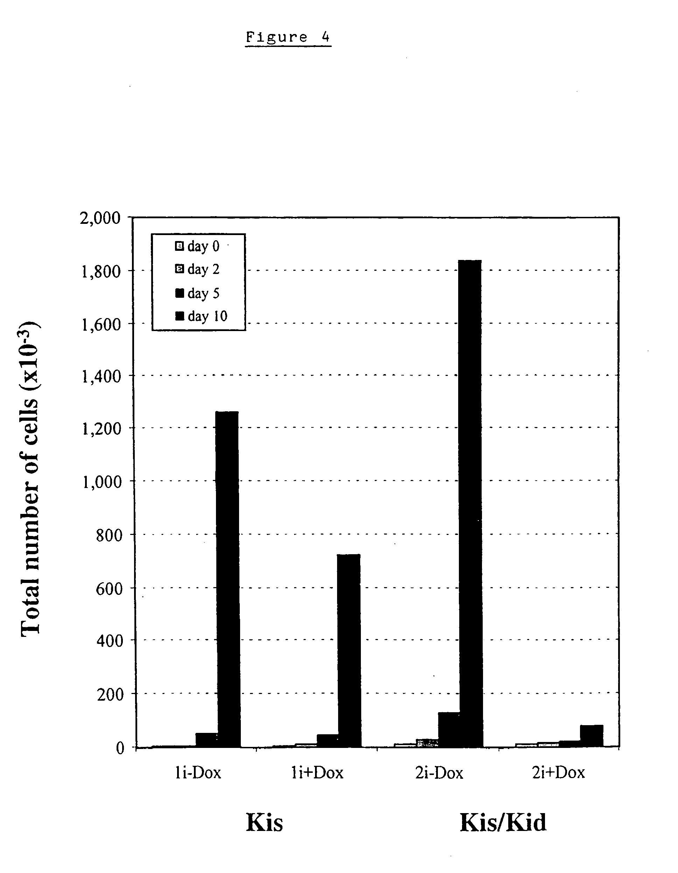

Figure 4 shows results of experiments in which *kis* expression was modulated by Doxycyclin in cultures of HeLa cells stably transfected with pNATHAli and pNATHA2i (Figure 3). Kid expression was controlled by CMV Pr which is unaffected by Doxycyclin.

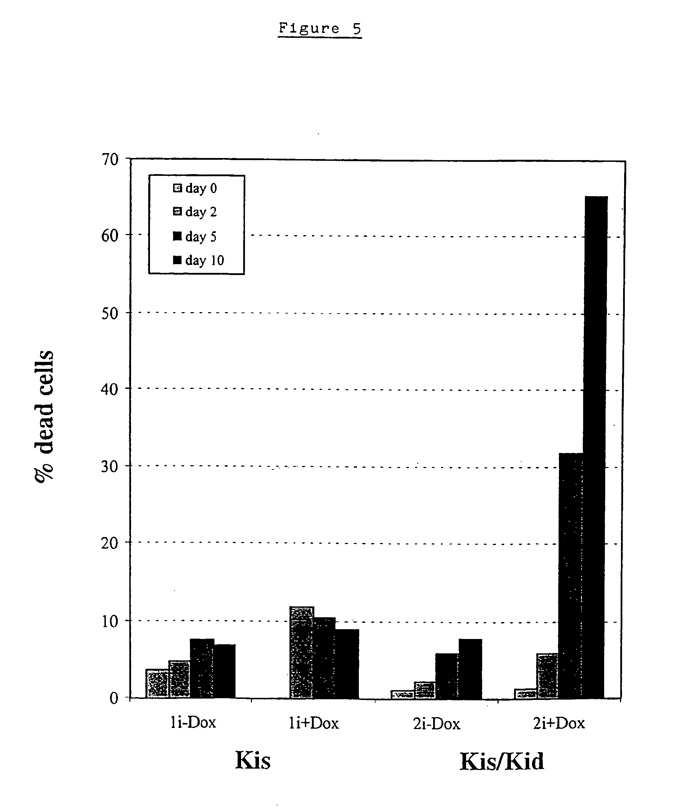

Figure 5 shows further results of experiments (numbers of dead cells) in which kis expression was modulated by Doxycyclin in cultures of HeLa cells stably transfected with pNATHAli and pNATHA2i (Figure 5). Kis expression was controlled by the Tet Pr which is repressed by Doxycyclin, while kid expression was controlled by CMV Pr which is unaffected by Doxycyclin.

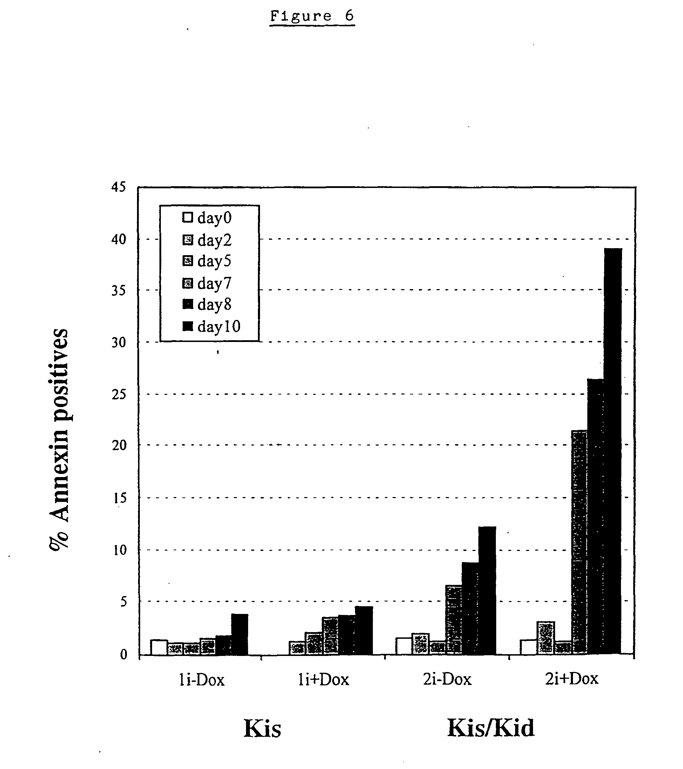

Figure 6 shows emergence of the apoptosis marker Annexin V in cells subject to the experiments of which results are shown in Figure 5, indicating the cell death caused by Kid to involve apoptosis.

According to one aspect of the present invention there is provided a composition comprising:
(i) the ParD kid toxin and ParD kis antitoxin, or
(ii) nucleic acid encoding the ParD kid toxin and ParD kis antitoxin,
for use in a therapeutic method of inhibiting cell proliferation and/or cell cycle progression carried out on a human or animal body, the method comprising providing within eukaryotic cells in the human or animal body the toxin and antitoxin, under appropriate control for selective cell cycle inhibition and/or killing of target cells.

According to a second aspect of the invention, use of a composition comprising:
(i) the ParD kid toxin and ParD kis antitoxin, or
(ii) nucleic acid encoding the ParD kid toxin and ParD kis antitoxin,
   in the manufacture of a medicament composition for use in a therapeutic method of inhibiting cell proliferation and/or cell cycle progression carried out on a human or animal body, the method comprising providing within eukaryotic cells in the human or animal body the toxin and antitoxin, under appropriate control for selective cell cycle inhibition and/or killing of target cells.

A third aspect of the invention is a method of inhibiting cell proliferation and/or cell cycle progression, the method comprising providing within eukaryotic cells the ParD kid toxin and ParD kis antitoxin under appropriate control for selective cell cycle inhibition and/or killing of target cells, wherein the cells are *in vitro* and/or are plant cells.

Some measure of control of toxin action is preferably employed in aspects of the present invention. Bacterial cell killing systems of use in the present invention naturally employ the PaD kis antitoxin. The present inventors have shown that both toxin and antitoxin are functional in various eukaryotic cells and that their respective activities can be controlled for selective inhibition of cellular proliferation or impedance of cell cycle progression, and/or induction of programmed cell death.

The bacterial cell killing system employed in the present invention comprises a toxin and an antitoxin which are both protein. Such a killing system is termed in the art a "proteic killer gene system" - Jensen & Gerdes, 1995, Mol. Microbiol. (1995) Jul 17(2): 205-10). The bacterial killer system used in the invention is an *E. coli* system.

Examples of bacterial killer systems (for references see Holcik and Iyer (1997), Microbiology, 143: 3403-3416 and references therein, and "Horizontal Gene Pool: Bacterial Plamids and Gene Spread" (1999), Ed. C M Thomas, Howard Academic Publishers, Chapter 2), include a bacterial plasmid-borne proteic killer gene system such as *ParD* (or R1 or homologues as discussed: above) *ccdA* (H or *let A*) of the F plasmid (antidote) and *ccdB* (G, *letB* or *letD*) toxin which acts by poisoning DNA-gyrase complexes (Jaffé, et al. (1985), Bacteriol, 163: 841-849) note that the mode of action of the ParD system is remarkably similar to that of the Ccd system), bacteriophage P1 toxic protein Doc with antidote Phd (Lehnherr, et al. (1993), Mol. Biol., 233: 414-428), *parDE* of plasmid RK2 (Roberts et al., 1994 J. Mol. Biol. 268, 27109-27117), with toxic protein ParE and antidote ParD, and *hig* of plasmid Rtsl (Tian et al., 1996, Biochem biophys Res Commun 220 280-284) with antidote *higA* to toxin *higB*.

Further examples of bacterial killer systems, where the natural antidote is an antisense RNA inlcude *parB* of plasmid R1 (Gerdes, et al,. (1990a), New Biol, 2: 946-956) with toxin Hok and antidote Sok (Thisted et al, 1994, EMBO J. 13, 1950-1959; *hok* mRNA is very stable but *sok* RNA decays rapidly), *srnB* (Onishi, (1975), Science, 187: 257-258) *flm* (Loh, et al. (1988), Gene, 66: 259-268) of the F plasmid and *pnd* of both Incl plasmid R483 and IncB plasmid R16 (Akimoto and Ohnishi (1982), Microbiol. Immunol., 26: 779-793), *relF* of the *E. coli* chromosomal *relB* operon (induction of the *relF* gene leads to the same physiological response as expression of the *hok* gene - Gerdes, et al. (1986a), EMBO J. 5: 2023-2029), *relB* homologues (Gronlund and Gerdes, 1999, J. Mol. Biol. 285, 1401-1415) and *Gef* (also chromosomal) which is structurally and functionally similar to the proteins encoded by *hok* and *relF* (Poulsen, et al. (1989), Mol. Microbiol. , 3: 1463-1472). Gef protein is toxic and regulated by antisense RNA Sof.

Further systems include *SegB* operon epsilon (antidote) and zeta (toxin) of pSM19035 and pDB101 (Ceglowski et al. (1993) Mol. Gen. Genet. 241 (5-6) : 579-85; Ceglowski et al. (1993) Gene 136 (1-2) : 1-12), *kicA* (antidote) and *kicB* (toxin) found in the *E. coli* chromosome (Feng, et al. (1984), Mol. Gen. Genet., 243 : 136-147), and the *kil*/*kor* systems carried by bacterial plasmids of the incompatibility groups P:and N. See Holcik and Iyer (Microbiology (1997) 143: 3403-3416) for examples and references. See also Jensen and Gerdes (Mol. Microbiol. (1995) 17 (2), 205-210) and Yarmolinsky (Science, (1995) 267, 836-837) for reviews of proteic killer gene systems, noted to have striking similarities in both structure and function.

The ParD Kid toxin is employed herein with the respective antidote, the ParD kis antitoxin. Additionally, the toxin may be employed with one or more other elements which inhibit or block its activity (which may be by inhibiting or blocking its production) as discussed.

Both toxin and antidote are introduced into eukaryotic cells under appropriate control for selective cell cycle inhibition and/or killing.

A method of the invention may include providing ParD kid toxin and ParD kis antitoxin to eukaryotic cells and, in target cells, removing or inhibiting the antitoxin to allow the toxin to work. Production or activity of antitoxin may be inhibited or blocked. This may be by provision of an appropriate stimulus, e.g. inducer or repressor molecule of a promoter controlling antitoxin production, or may occur under conditions prevailing in target cells. As discussed below, the presence of a different form of a protein such as p53 in target cells vs. non-target cells (e.g. for p53 tumour and non-tumour cells) can be employed as a controlling stimulus. An inducer or repressor molecule may be delivered to target cells to inhbit or block antitoxin and/or upregulate toxin.

Generally, the cell killing system is provided to cells by means of nucleic acid encoding the relevant components and, where applicable, control elements (discussed further below).

Control elements may include any one or more of those available in the art allowing for selective variation of the ratio of toxin versus antitoxin. Examples include an inducible, repressible or constitutive promoter, antisense constructs and their activator or repressors, ribozymes, splicing sequences and splicing factors, recombination systems (e.g. Cre-lox or FLP); wild-type or modified Internal Ribosome Entry Sites (IRES) (Schmid and Wimmer (1994), Arch. Virol. Suppl., 9: 279-89; Borman, et al. (1994), EMBO J., 1:13(13): 3149-57)and IRES inhibitors such as a yeast RNA that inhibits entry of ribosomes at some IRES (Das, et al. (1996), J. Virol., 70 (3): 1624-32; Das; et al. (1998), J. Virol., 72(7): 6638-47; Das, et al. (1998), Front Biosci., 1:3: D1241-52; Venkatosan, et al. (1999), Nucleic Acids Res. 15: 27 (2): 562-72), elements that allow transcriptional interference between promoters (Greger and Proudfoot (1998), 17:17 (16) : 4771-9 ; Eggermont and Proudfoot (1993), EMBO J., 12(6): 2539.-48 ; Bateman and Paule (1998), Cell, 23:54(7): 985-92; Ponnambalam and Busby (1987), FEBS Lett., 9:212(1): 21-7; Greger, et al. (1998), Nucleic Acids Res.,..1:26(5): 1294-301), inteins (Chong et al. (1996) J. Biol Chem 271 (16) : 22159-68).

A eukaryotic vector may be provided, comprising nucleic acid encoding a toxin or cell killing system, as disclosed. Such a vector may be used to provide the toxin or cell killing system to eukaryotic cells.

Nucleic acid encoding a bacterial toxin and antidote may be provided as part of a vector or vectors suitable for transformation of eukaryotic cells. Preferably the vector is suitable for transformation of target cells, for instance it may be suitable for transformation of plant cells (e.g. an *Agrobacterium* vector). Where two components of a bacterial killing system are employed, or a toxin is employed and a specifically designed regulatory element is employed (e.g. antisense or ribozyme), preferably both components and regulatory elements for control of expression are provided on the same vector, but may be provided on separate vectors. Either or both of the encoding nucleotide sequences may be under transcriptional control of a specific and/or regulatable promoter. Toxin- and antidote- encoding sequences may be provided in a "tail-to-tail" or inverted orientation, or in a head-to-tail orientation.

Advantageously, for example in yeast, nucleic acid encoding toxin is provided on a multicopy plasmid, such as, for yeasts 2*µ* (Christianson, et al. (1992), Gene, 2:110 (1) : 119-22), for mammalian cells a vector including oriP from Epstein Barr Virus (that may be accompanied by the initiator protein EBNA1 Kirchmaier and Sugden (1995), J. Virol., 69(2): 1280-3; Wendelburg and Vos (1998), Gene Ther., Oct:5(10): 1389-99), or the origin from the Bovine Papilloma Virus (that needs also two virus encoded proteins to be active (Piirsoo, et al. (1996), EMBO J., 2 :15 (1) : 1-11), or a viral vector.

Monocopy vectors useful in the present invention include, for yeast, ARS1 and ARSH4/CEN6 (Sikorski and Hieter (1986), Genetics, 122(1): 19-27; Mumberg, et al. (1995), Gene, 14:156(1): 119-22).

Suitable vectors can be chosen or constructed, containing appropriate regulatory sequences, including-promoter sequences, terminator fragments, polyadenylation sequences, enhancer sequences, marker genes and other sequences as appropriate. Vectors may be plasmidic and/or viral and maintained in cells as episomes or integrated into the genome. For further details see, for example, Molecular Cloning: a Laboratory Manual: 2nd edition, Sambrook et al., 1989, Cold Spring Harbor Laboratory Press. Many known techniques and protocols for manipulation of nucleic' acid, for example in preparation of nucleic acid constructs, mutagenesis, sequencing, introduction of DNA into cells and gene expression, and analysis of proteins, are described in detail in Short Protocols in Molecular Biology, Second Edition, Ausubel et al. eds., John Wiley & Sons, 1992.

The ParD kid toxin and ParD kis antitoxin may be provided in accordance with the present invention to a eukaryotic cell selected from mammalian, human or non-human such as rabbit, guinea pig, rat, mouse or other rodent, cat, dog, pig, sheep, goat, cattle or horse, bird, such as a chicken, yeast, fungi, amphibian, fish, worm, and plant Plants which may be employed in the present invention have been noted already above.

A eukaryotic cell may be provided containing nucleic acid encoding a bacterial toxin and/or antidote or cell killing system as disclosed herein, under appropriate regulatory control. The nucleic acid may be integrated into the genome (e.g. chromosome) of the cell. Integration may be promoted by inclusion of sequences which promote recombination with the genome, in accordance with standard techniques. The nucleic acid may be on an extra-chromosomal vector within the cell.

A method of the invention may include introducing the nucleic acid into a eukaryotic cell. The introduction, which may (particularly for *in vitro* introduction) be generally referred to without limitation as "transformation", may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus.

The introduction may be followed by causing or allowing expression from the nucleic acid, e.g. by culturing cells (which may include cells actually transformed although more likely the cells will be descendants of the transformed cells) under conditions for expression of one or more components of the system, so that an encoded product is produced. The conditions may provide for cell killing (or inhibition of cell cycle progression, cell growth or proliferation, etc.), and/or neutralisation of the toxic effect when appropriate.

Introduction of nucleic acid may take place in vivo by way of gene therapy, as discussed below. A cell containing nucleic acid encoding a system according to the present invention, e.g. as a result of introduction of the nucleic acid into the cell or into an ancestor of the cell and/or genetic alteration of the sequence endogenous to the cell or ancestor (which introduction or alteration may take place *in vivo* or ex *vivo),* may be comprised (e.g. in the soma) within an organism which is an animal, particularly a mammal, which may be human or non-human, yeast, fungal, amphibian, fish, worm or plant, with examples noted already above. Genetically modified or transgenic animals, birds or plants comprising such a cell may also be provided.

Thus, there may be provided a non-human animal with nucleic acid encoding a bacterial cell killing system (as disclosed) within its genome. The animal may be rodent, e.g. mouse, and may provide an animal model for investigating aspects of cell cycle control, cell killing, apoptosis or other cellular process, and drug screening.

A plant with nucleic acid encoding a bacterial cell killing-system within its genome, a plant cell (which may be in culture, e.g. callus culture, or comprised in a plant or plant part), or a plant part (e.g. fruit, leaf, seed or other propagule) may also be provided.

For generation of plant material comprising nucleic acid encoding a bacterial cell killing system as disclosed, any appropriate means of transformation may be employed. Agrobacterium transformation is widely used by those skilled in the art to transform both dicotyledonous and monocotyledonous species. Microprojectile bombardment, electroporation and direct DNA uptake are preferred where Agrobacterium is inefficient or ineffective. Alternatively, a combination of different techniques may be employed, e.g. bombardment with Agrobacterium coated microparticles or microprojectile bombardment to induce wounding followed by cocultivation with Agrobacterium. Following transformation, a plant may be regenerated, e.g. from single cells, callus tissue or leaf discs, as is standard in the art. Almost any plant can be entirely regenerated from cells, tissues and organs of the plant.

Where a bacterial cell killing toxin is employed there are various strategies for controlling its activity. Generally, the relevant antidote is employed to neutralise the toxic effect unless and until the toxicity is desired. Thus, for example, both toxin and antidote may be expressed in normal cells, with antidote production being down-regulated in target cells (e.g. tumour cells). Toxin production may be down-regulated in normal cells and/or upregulated in target cells. Antidote production may be upregulated in normal cells and/or downregulated in target cells.

Upregulation of toxin and/or antidote production, depending on context, may be achieved by a number of means. A preferred approach is to employ a promoter or other regulatory element that is inducible under certain conditions, allowing for control of expression by means of application of an appropriate stimulus.

A tumour specific promoter such as telomerase RNA promoter may be employed. In plants nematode inducible promoters such as TobRB7 (Opperman et al., Science 263: 221-223) and PRP1 (pathogenesis related protein - see e.g. Payne et al. (1989) Plant Molecular Biology 12: 595-596; also Memelink et al. (1990) Plant Molecular Biology 14: 119-126 and Payne et al. (1990) Proc. Natl. Acad. Sci. USA 87: 98-102) may be employed.

Downregulation of toxin and/or antidote, again depending on context, may also be achieved by means of regulation of gene expression using an appropriate promoter or other regulatory element, including a repressor element, such as Tet Pr. Other approaches which may be employed include antisense regulation and ribozymes (discussed further below).

Thus, for example, antidote production may be downregulated by production of an antisense transcript or ribozyme. The antisense transcript or ribozyme may be produced on application of an appropriate stimulus, and may be be produced by expression from a sequence under transcriptional control of an inducible promoter or other regulatory element.

By "promoter" is meant a sequence of nucleotides from which transcription may be initiated of DNA operably linked downstream (i.e. in the 3' direction on the sense strand of double-stranded DNA).

"Operably linked" means joined as part of the same nucleic acid molecule, suitably positioned and oriented for transcription to be initiated from the promoter. DNA operably linked to a promoter is "under transcriptional initiation regulation" of the promoter.

The term "inducible" as applied to a promoter is well understood by those skilled in the art. In essence, expression under the control of an inducible promoter is "switched on" or increased in response to an applied stimulus (which may be generated within a cell or provided exogenously). The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus. The preferable situation is where the level of expression increases upon application of the relevant stimulus by an amount effective to provide the desired result. Thus an inducible (or "switchable") promoter may be used which causes a basic level of expression in the absence of the stimulus which level is too low to bring about the desire result (and may in fact be zero). Upon application of the stimulus, expression is increased (or switched on) to a level which brings about the desired result.

Examples of inducible-promoters for use in aspects of the present invention include a minimal promoter, such as CMV minimal promoter, fused to an enhancer for wild-type p53 activation or mutant p53 repression whether bearing the consensus DNA binding sequence for wild-type p53, e.g. fragment A (Kern, et al. (1991), Science, 252(5013): 1708-11) or CON (Chen, et al. (1993), Oncogene, 8(8): 2159-66), or not, e.g. HIV 1-LTR (Subier, et al. (1994), J. Virol., 68(1): 103-10; Gualberto and Baldwin (1995), J. Biol. Chem., 25: 270(34): 19680-3; Sawaya, et. al. (1998), J. Biol. Chem., 7:273(32): 20052-7, inducible or repressible promoters such as Tet Pr as discussed and galactose activatable GAL10-CYC1. For plants suitable promoters include the inducible GST-II promoter from maize (Jepson et al. (1994). Plant Molecular Biology 26:1855-1866), alcohol inducible promoter (e.g. alcr - see e.g. Gatz (1998) Nature Biotechnology 16: 140), and the Cauliflower Mosaic Virus 35S (CaMV 35S) gene promoter that is expressed at a high level in virtually all plant tissues (Benfey et al, (1990) EMBO J 9: 1677-1684).

As noted, toxin production may be downregulated in non-target cells by employing elements for control of expression. Alternatively or additionally downregulation may employ antisense nucleic acid or ribozymes. One or more of these approaches may be: employed in addition to use of antitoxin to neutralise toxin activity. Antitoxin production itself may be controlled, as discussed.

In a preferred approach selectivity for expression within target cells of the toxin in accordance with the present invention is effected by a combination of (i) up-regulation of toxin production in target cells and (ii)- down-regulation of toxin production in non-target cells and/or neutralisation of toxin activity in non-target cells (for instance by upregulation of antidote production in non-target cells). Effect (i) will mediate the desired activity in target cells, while effect (ii) will reduce the extent of "leaky" expression of that activity in non-target cells.

Where target cells are tumour cells, and non-target cells are normal cells, advantage can be taken of the fact that p53 is mutated or its function inactivated in a large proportion of tumours. The p53 protein is a transcriptional activator in normal cells but is present in mutant form in a substantial proportion (40-80%) of human tumours. Even in tumours in which the p53 sequence is wild-type, its normal function in cell cycle control, DNA repair, differentiation, genome plasticity or apoptosis may be abrogated, for instance by interaction with cellular protein (e.g. mdm2) or oncoviral protein (e.g. SV40 T antigen, human papillomavirus E6 protein, adenovirus E1B protein, hepatitis B virus X protein, and Epstein-Barr BZLF-1 protein), or by being sequestered in the cytoplasm, where the p53 protein is non-functional.

Accordingly, production of the antidote (or antisense RNA or a ribozyme directed against the toxin) may be controlled by a promoter whose function is upregulated by wild-type p53 in normal cells but not by mutant p53 in tumour cells. Wild-type p53 protein binds to two copies of the consensus sequence 5'-PuPuPuC (A/T) (A/T) GpyPyPy-3' (SEQ ID NO. 1) and thereby transactivates the level of transcription from an operably linked promoter. Most of the mutations in the p53 gene lead to abrogation of the sequence-specific transcriptional activating function.

Production of the toxin may be controlled by a promoter whose function is suppressed by wild-type p53 protein in normal cells, but is not suppressed or is even upregulated by mutant p53 protein, e.g. hsp70 promoter, mdm2 promoter and Others. See for example "The Oncogene and Tumour Suppressor Gene Facts Book", Robin Hesketh, Academic Press, Second Edition (1997) Chapter p53, pages 446-463 and references therein.

The promoters of a number of cellular genes are negatively regulated by wild-type p53, include basic FGF (also activated by mutant p53), Bcl-2, human interleukin 6 and PCNA. Again, see "The Oncogene and Tumour Suppressor Gene Facts Book", Robin Hesketh, Academic Press, Second Edition (1997) Chapter p53, pages 446-463 and references therein for examples. Viral promoters inhibited by wild-type p53 and in some cases activated by mutant versions are referenced in Deb et al. (1992) J. Virology, 66(10): 6164-6170.

Accordingly, such a promoter or a binding site for wild-type p53 from such a promoter may be operably linked to nucleic acid encoding the toxin. In normal cells, wild-type p53 protein suppresses production of the toxin. However, in tumours where p53 is not functional and does not bind its binding site in the promoter, toxin production is derepressed.

Similarly, a response element which is activated by mutant p53 but not wildtype, such as from HIV1-LTR DNA sequences, may be employed to provide for upregulation of toxin in tumour cells, or downregulation of antidote where a third component is employed to control antidote production in tumour cells. An element activated by mutant p53 element (for example) may by used to upregulate an antisense RNA, ribozyme or other factor which downregulates antidote production in tumour cells.

In non-target cells production of toxin may be inhibited by using appropriate nucleic acid to influence expression by antisense regulation. Such approaches may be used to downregulate antidote production in target cells. The use of anti-sense genes or partial gene sequences to down-regulate gene expression is now well-established. Double-stranded DNA is placed under the control of a promoter in a "reverse orientation" such that transcription of the "anti-sense" strand of the DNA yields RNA which is complementary to normal mRNA transcribed from the "sense" strand of the target gene. The complementary anti-sense RNA sequence is thought then to bind with mRNA to form a duplex, inhibiting translation of the endogenous mRNA from the target gene into protein. Whether or not this is the actual mode of action is still uncertain. However, it is established fact that the technique works.

Another possibility is that nucleic acid is used which on transcription produces a ribozyme, able to cut nucleic acid at a specific site - thus also useful in influencing gene expression. Background references for ribozymes include Kashani-Sabet and Scanlon (1995). Cancer Gene Therapy, 2, (3) 213-223, and Mercola and Cohen (1995). Cancer Gene Therapy 2,(1) 47-59.

Thus, an antisense RNA or ribozyme directed against toxin expression may be used to downregulate production in non-target cells. Antisense RNA or ribozyme production may be placed under control of a regulatable promoter so that such production can be downregulated in target cells (for instance by means of a p53 element as discussed above).

An approach to downregulating toxin production in non-target cells (e.g. normal cells), and/or upregulating toxin production in target cells (e.g. tumour cells), may be instead of or in addition to regulating antidote production.

A further possibility is to use antisense RNA or a ribozyme or other approach to downregulate antidote production in target cells. Upregulating production in target cells of an antisense RNA or ribozyme against antidote may be used to reduce levels of antidote in target cells and thereby increase toxin activity in those cells.

Control of translation may be employed, for instance by means of an internal ribosome entry sequence (IRES) which may be controled using a RNA from yeast (Das, et al. (1996), J. Virol., 70(3): 1624-32; Das, et al. (1998), J. Virol., 72(7): 6638-47; Das, et al. (1998), Front Biosci., 1:3: D1241-52; Venkatosan, et al. (1999), Nucleic Acids Res. 15:27(2): 562-72) or other that inhibit ribosome assembly at the IRES.

In further embodiments, the killing system, toxin and/or antidote or other inhibitor is provided to cells as protein, for instance by direct injection into target cells, such as in a tumour. In one embodiment, a carrier molecule is employed to facilitate uptake by cells, e.g. a 16 aa peptide sequence derived from the homeodomain of *Antennapedia* (e.g. as sold under the name "Penetratin"), which can be coupled to a peptide via a terminal Cys residue. The "Penetratin" molecule and its properties are described in WO 91/18981.. Another example is VP22 (Elliott and O'Hare (1999) Gene Ther 6(1): 149-51; Dilber et al. (1999) Gene Ther 6(1): 12-21; Phelan et al. (1998) Nat Biotechnol 16 (5) : 440-3).

Expression and purification of a toxin antidote is straightforward. However, the toxic nature of a toxin such as the Kid protein makes these more difficult to over-express and purify. However, appropriate strategies are available or can be devised by those of ordinary skill in the art. With reference to Kis/Kid, in absence of a Kid resistant genetic background, the Kis antidote may be co-expressed at the same time in the Kid overproducer strains. The tight interaction that takes place between both proteins to generate a neutralised complex allows purification from a whole bacterial extract and separation of the components afterwards by chaotropic denaturation and further chromatographic purification and renaturation of the toxic component. A bacterial one-or two- affinity chromatography-based approach has been designed to purify Kid and Kid variants in high amounts and a refolding protocol has been standardised to obtain active, pure and concentrated preparations of the parD system toxin. See the experiments described below. Such an approach may be used to purify other toxic components of different stability systems.

A composition comprising nucleic acid, protein or cells according to the present invention may comprise at least one additional component, such as a pharmaceutically acceptable diluent, vehicle or carrier, or a solvent or carrier for delivery to the target organism, e.g. plant. Nucleic acid, proteins, cells and compositions as-disclosed herein may be used in a method of treatment of the human or animal body by way of therapy, e.g. for treatment of tumours, cancer, psoriasis, arteriosclerosis, any other hyper-proliferative disorder, or other disorder. Nucleic acid, protein, cells and compositions may also be used in the manufacture of a medicament for such treatment, and methods of treatment comprising administration of a medicament or pharmaceutical composition to a eukaryote. Methods may comprise treating eukaryotic cells with nucleic acid, protein, cells or compositions as disclosed herein. The eukaryotic cells may be for example any yeast, mammalian, plant, amphibian, avian, fish or worm. Cells to be treated may be *in vitro* or in culture , or may be comprised in a mammalian (e.g. human) body or plant or plant part (e.g. fruit, leaf, seed or other propagule).

Compositions, cells and methods as described herein may be used in methods in which expression of a desired gene is targeted to desired cells, e.g. tumour cells as opposed to non-tumour cells. Such methods may be performed *in vivo* (e.g. by way of treatment of a human:or animal body for therapeutic purposes), *ex vivo* (e.g. on cells removed from a human or animal body, prior to return of the cells to the body) or *in vitro.* Compositions and cells may be used in the manufacture of a medicament for treatment in which expression of a desired gene is targetted to target cells (e.g. tumour cells). Nucleic acid constructs may form part of a viral vector, for instance a viral vector engineered to be suitable for administration to an individual, such as a human, and preferably additionally tumour targetting.

Compositions provided may be administered to individuals. Administration is preferably in a "therapeutically effective amount", this being sufficient to show benefit to a patient. Such benefit may be at least amelioration of at least one symptom. The actual amount administered, and rate and time-course of administration, will depend on the nature and-severity of what is being treated. Prescription of treatment, e.g. decisions on dosage etc, is within the responsibility of general practitioners and other medical doctors.

A composition may be administered alone or in combination with other treatments, either simultaneously or sequentially dependent upon the condition to be treated.

Pharmaceutical compositions according to the present invention, and for use in accordance with the present invention, may comprise, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration.

Experimental support for the present invention will now be described by way of illustration. Various additional aspects and embodiments of the present invention will be apparent to those skilled in the art.

"Comprising" herein is used with the meaning of "including", that is permitting the presence of one or more additional components or features.

### EXAMPLE 1

### Effect of expression of the parD system in Saccharomyces cerevisiae

Several plasmids with different constitutive and/or regulatable promoters were tested for their ability to express both components of the parD system separately in a controlled fashion. The results were similar with all the promoters used. In addition to the promoters used as described in detail in the following experiments, the inventors performed experiments using the ADH5 promoter (constitutive; Mumberg, et al. (1995), Gene, 14:156 (1) : 119-22) for kis and GAL10-CYC1 (galactose activatable Guarente, et al. (1982), Proc. Natl. Acad. Sci. USA, 79 (23) : 7410-4) for kid.

Antidote transcription in *S. cerevisiae* was controlled by a promoter induced by Cu²⁺, while the toxin transcription was controlled by a different promoter repressed by methionine. With that purpose, the former was cloned in a monocopy plasmid (ARSH4/CEN6 origin of replication) and the latter was cloned in a multicopy plasmid (2µ origin of replication) that confer auxotrophy for leucine and tryptophan respectively to a transfected yeast (Figure 1).

Using a multicopy plasmid for the toxin expression has two advantages: first, it reduces the possibility of selecting cells that have inactivated that protein by mutation of its DNA, as each cell should have to inactivate all the copies (10-30 molecules per haploid genome for a 2µ origin harbouring plasmid) of the kid gene present in each cell. Mutation of that gene in growth conditions in which the system is inactivated by expression of the antidote is unlikely as in that situation there is no selective pressure for the cells in order to accumulate mutations. This is verified by the fact that induction of the system exerts a clear inhibitory effect over *S. cerevisiae* growth (see below). Secondly, this approach showed that it is also possible to regulate the amount of mRNA of each component of the system by increasing or decreasing the number of encoding DNA molecules for each one (i.e. their copy number) without modifying the strength of their promoters. This allows greater flexibility in the design of systems in eukaryotes, e.g. for yeast, anti-fungals etc.

Different *S. cerevisiae* strains transfected with kis+/kid+, kis+/kid- or kis-/kid- plasmids were grown in liquid selective medium (-Leu/-Trp) in presence of amounts of Cu²⁺ and methionine that maintain the parD system in an inactivated state, before plating different serial dilutions of these cultures in solid media with a constant amount of methionine to give a constant expression of Kid (if any) in all the cases, but reduced concentrations of Cu²⁺ to decrease expression of its antidote from plate to plate. Kis and kid harbouring cells were not able to grow in media without Cu²⁺ and this effect is decreased as Cu²⁺ concentration increases until it reaches approximately the same rate of growth as wild type (kis-/kid-) cells. In contrast, both kis+/kid- and wild type (kis-/kid-) cells were able to grow normally under all circumstances tested.

This experiment demonstrated that Kid and Kis are active as a toxin and its antidote respectively in yeast and that it is possible to regulate their activity (and thus parD activation or inactivation) by means of transcriptional control of its components in S. *cerevisiae.* It also provides indication that antidote expression alone has no side effects and that the biological process inhibited by the parD toxin is conserved among distantly evolved organisms.

### EXAMPLE 2

### Effect of the proteins of the parD system in Xenopus laevis

Two cell stage embryos from *Xenopus laevis* were injected at the animal pole of one of the blastomers either with Kis, Kid, both or none of them (buffer) and its effects on subsequent cell divisions were followed along time. Kid injected embryos only divided correctly in the non-injected blastomer, while Kis-, Kis/Kid- and buffer- injected embryos blastomers progressed in all cases in the same way as the non-injected ones along the embryonic development stages followed in the experiment (at least until mid blastula transition, MBT).

This experiment further indicates that eukaryotic cell cycle progression is severely affected by non-neutralised Kid protein and suggests that this effect is not exerted in any of its gap phases (G1 or G2) as they are not present in the first stages of *X. laevis* development. It also confirms that it affects a conserved biological process among distant species and offers some clues related to the possible mechanism of action of Kid (as *X. laevis* embryonic replication does not require specific DNA sequences to initiate). The fact that progression through the cell cycle of the non-injected blastomers in the Kid injected embryos is not affected at all, together with the lack of effects in both halves of the other injected embryos (Kis, Kis/Kid and buffer), clearly indicates that the Kid gene product is the responsible for that phenotype in eukaryotes and that the Kis gene product is responsible for its neutralisation and has no side effects *per se*, when used alone.

### EXAMPLE 3

### Effect of the parD system in human cells

The above results from yeast and amphibians show that Kid is able to impede cell cycle progression through the cell cycle in eukaryotes in a controlled fashion and that it is possible in these organisms to substitute the prokaryotic regulatory circuits that maintain the parD system in a silent state under desired conditions by modulating transcription of both the antidote and the toxin with different promoters.

For experiments in human cells a set of plasmids named pNATHA (for plasmids with Neutralisable Activity that Triggers HeLa Apoptosis) was constructed. Their mechanism of action is based in the observation that in HeLa Tet Off cells a Cytomegalovirus Early promoter (CMV Pr) maintains a constant level of transcription of a reporter gene independently of the presence or absence of Tetracyclin (or Doxycyclin) in the culture medium. On the other hand, using the same cell line, a Tetracyclin regulatable promoter (Tet Pr) can decrease the level of transcription of that reporter gene by more than three orders of magnitude upon addition of the transcriptional regulator. In the induced state (i.e. in absence of Dox) Tet Pr directed transcription of the reporter gene is almost two orders of magnitude higher than that of the same reporter gene under control of the CMV Pr. In the uninduced state (i.e. in the presence of Dox), the latter transcribes almost two orders of magnitude more efficiently than the former (Figure 2).

This transcriptional behaviour offers a window that can be used to construct the pNATHA plasmids, in which both *kis* and *kid* genes are contained in the same DNA molecule, the antidote mRNA synthesis controlled by the Tet repressible promoter and the toxin messenger levels controlled by the CMV constitutive one. Both cassettes contained Kis and Kid were cloned in either direct or inverted orientations (Figure 5). Toxin and antidote can be cloned in a tail-to-tail or tail-to-head orientation as convenient and to take advantage of transcriptional interference under appropriate control Both may be part of the same transcriptional unit if an IRES is placed between the coding sequences.

Additional variants of both the antidote and the toxin were tested in HeLa cells, after verifying their wild type-like activity in vivo in *E. coli.* A Nuclear Localisation Signal (NLS) was fused to Kid and Kis to test if it would confer a more efficient effect (if any in human cells) both impeding cell cycle progression or neutralising that impedance, respectively.

All pNATHA were stably transfected in a HeLa Tet Off cell line. The *in vivo* effect of both components of the parD system on these cells was analysed before and after addition of Doxycyclin to the different cultures. The first observation of this set of experiments is that, again, after induction of the system, cell growth rate is severely inhibited in HeLa kis+/kid+ and nlskis+/kidnls+ cells. This suggests two different things: first, that immediate transport of the toxin into the nucleus (verified by confocal microscopy of Kid immunostained samples) does not impede its toxic effect, indicating the probable nuclear localisation of its cellular target(s); and second, that the wild type components of the parD system are as active as NLS-fused ones in HeLa cells, which indicates either that entry into the nucleus is not impeded for the wild type proteins, and/or that inactivation of the cellular target(s) by Kid can occur in the cytosol. After one or two days growing in presence of Doxycyclin, and up to ten days of treatment, an induced state of parD is detectable, as kis+/kid+ cells have increased doubling time, compared to kis+/kid- transfectants or to kis+/kid+ cells grown in absence of Doxycyclin (Figure 4). It should be noted that as only kis transcription is being modulated directly, while maintaining constant level of kid, the rate of growth for those kis+/kid+ stabilised transfectants is lower than that of their kis+/kid-counterparts in the same conditions. This could be due to a slight escape of the system at the level of its neutralisation ability if kid transcription is not reduced selectively at the same time.

The results showed progressive reduction of cell doublings of kis+/kid+ stable transfectants upon continued exposure to Doxycyclin (i.e. to non-neutralised toxin). The inventors were interested in whether it would be possible to provide a cytostatic and/or cytotoxic effect.

Percentage of dead cells was determined after treatment with sub-lethal doses of Doxycyclin of the different stable transfectants analysed previously. As indicated before, kis+/kid- HeLa cells showed an exponential growth rate along time in both presence and absence of Doxycyclin. On the contrary, kis+/kid+ HeLa cells showed an exponential cell growth rate only when antidote transcription was maintained (i.e. in absence of Doxycyclin) but not in the opposite case, in which they reduced continuously their number of doublings (Figure 5). It should be noted though that growth rate was reduced for kis+/kid- HeLa cells grown in presence of Doxycyclin compared to that of the same stabilised cell line grown in its absence. This effect may be due to long exposure to Doxycyclin even at sub-lethal doses and, in any case, it does not lead to cell death. When dead cells were counted for all the samples, kis+/kid+ HeLa cells growing in presence of Doxycyclin (i.e. in presence of non-neutralised toxin) showed a 32% and 65% of dead cells at days five and ten of treatment, respectively, while all the other samples did not show more than 9% even upon ten days of treatment (Figure 5). Annexin V (i.e. an early apoptotic marker) staining of the different samples analysed, demonstrates that the observed cell death in kis+/kid+ non-neutralised HeLa cell line was due to activation of apoptosis (Figure 6).

### MATERIALS AND METHODS

### Saccharomyces cerevisiae

### Plasmids

Oligonucleotides XholKis (5'CCGCTCGAGATGCATACCACCCGACTG3, - SEQ ID NO. 2) and KisNcol (5'CATGCCATGGTCAGATTTCCTCCTGACCAG3' - SEQ ID NO. 3) were used to amplify the kis coding region by PCR from a mini-R1 derivative. The amplified product was digested with Xhol and Ncol and cloned in the plasmid pSAL1 to construct pSAL1Kis (Mascorro-Gallardo, et al. (1996), Gene, 172(1): 169-70). In a similar way, oligonucleotides ATGKid (5'ATGGAAAGAGGGGAAATCTG3' - SEQ ID NO. 4) and KidEcoRI (5'CGGAATTCCCCATGTTCAAGTC3' - SEQ ID NO. 5) were used to amplify the kid coding region using the same template and the product obtained was digested with EcoRI and cloned in the plasmid p424Met25 (Mumberg, *et al*. (1994), *Nucleic Acids Res.,* 25:22(25): 5767-8) digested with SmaI and EcoRI to construct the plasmid p424Met25Kid. This plasmid was amplified in a bacterial strain that overproduces Kis at the same time to abolish selection of inactivated mutants during the cloning process.

### In vivo assay

*Saccharomyces cerevisiae* strain W303α (MAT α, ade2-1, trpi-1, can1-100, leu2-3, 112, his3-11, ura3, psi+) was transformed with plasmids pSALl and p424Met25 (null), pSAL1Kis and p424Met25 (kis+/kid-) and pSAL1Kis and p424Met25kid (kis+/kid+). These cells were grown in selective medium supplemented with 500 µM of methionine and 200 µM of SO₄Cu to maintain the kis and kid promoters in an activated and repressed state respectively. The cultures were allow to grow until mid-log phase and then a 3 µl drop of dilutions of each culture containing 15000, 1500 or 150 cells was posed in agar plates made of selective medium supplemented with 200 µM of methionine to maintain a constant expression level of the kid gene and 0, 1, 5, 10, 20, 40, 80, 100 and 200 µM of SO₄Cu to increase the expression level of the kis gene. The plates were incubated 48 hours at 30°C and the growth rate of each culture was analysed afterwards on each plate.

### Xenopus laevis

### Kis and Kid overproducers

### MBPKis overproducer

Oligonucleotides ATGKis (5'ATGCATACCACCCGACTG3' - SEQ ID NO. 6) and KisEcoRI (5'TCGGAATTCAGATTTCCTCCTG3' - SEQ ID NO. 7) were used to amplify kis by PCR using a mini-R1 plasmid as template. The amplified product was digested with EcoRI and cloned in pMAL-c2 plasmid (Mumberg, et al. (1994), Nucleic Acids Res., 25:22(25): 5767-8) between the Xmnl and EcoRI sites to obtain the MBP- (Maltose Binding Protein) Kis overproducer.

### HisKisKid overproducer

Oligonucleotides NdeIkid (5' GGAATTCCATATGCATACCACCCGACT3' - SEQ ID NO. 8) and kisBamH1 (5'CGGGATCCTCAAGTCAGAATAGT3^{,} - SEQ ID NO. 9) were used to amplify the coding regions of kis and kid in tandem from a mini-R1 derivative. The product of PCR was digested with NdeI and BamHI and cloned in pET15b (Invitrogen) between these sites. The resultant plasmid was disgested with NcoI and BamH1 and the DNA fragment codifying for Hiskiskid was purified and subcloned between these same sites in pRG-recA-NHis (Giraldo, et al. (1998), EMBO J., 3:17(15): 4511-26).

### Protein purification

### MBPKis purification

Kis protein was purified as a fusion with the Maltose Binding Protein (MBP). *Escherichia coli* strain DH5α transformed with the plasmid pMBPKis was inoculated in 2 L of LB medium plus ampicillin (100 *µ*g/ml) at 0.04 units of Abs₆₀₀ₙₘ and grown with shaking at 37°C until 0.4 units of Abs₆₀₀ₙₘ were reached. MBPKis expression was induced then by addition of IPTG 100 *µ*M to the culture medium. Cells were grown for 4 hours at 37°C and then pelleted in a GS3 rotor and resuspended in 10 ml lysis buffer (20 mM Tris-HC1 pH 8.0, 150 mM NaCl) and frozen in liquid nitrogen. After thawing cells, 2 mg of lysozyme was added to the suspension of cells and lysis was completed by incubation at 37°C for about 10 minutes, with cooling on ice every 3 minutes. A soluble fraction was obtained by addition of 40 ml of buffer 20 mM Tris-HCl pH 8.0, 600 mM NaCl and centrifugation at 30 Krpm at 4°C during 45 min in a 65 Ti rotor. MBPKis protein was purified by affinity chromatography through an amylose resin (BioLabs) following the manufacturers instructions in buffer 20 mM HEPES pH 7.5, 100 mM KC1, 1 mM DTT and 10% of ethyleneglycol. MBPKis fractions were pooled and purity and concentration of the protein were determined by coomassie staining on a SDS-PAGE gel and by spectrophotometric analysis, respectively. Fractions were stored at -80°C.

### Kid purification

*Escherichia coli* strains C600 or TG1 transformed with the overproducer pRGΔHisKisKid were grown in 2 L of LB medium plus ampicillin (100 µg/ml ) at 0.04 units of Abs₆₀₀ₙₘ, and grown with shaking at 37°C until 0.4 units of Abs₆₀₀ₙₘ were reached. HisKis and Kid expression was induced then by addition of 25 µg/ml of nalidixic acid to the culture medium. Cells were grown for 4 hours at 37°C and then.pelleted in a GS3 rotor and resuspended in 10 ml lysis buffer (20 mM Tris-HCl pH 8.0, 150 mM NaCl) and frozen in liquid nitrogen. After thawing, 2 mg of lysozyme was added to the suspension of cells and lysis was completed by incubation at 37°C. A soluble fraction was obtained by addition of 40 ml of buffer 20 mM Tris-HCl pH 8.0, 600 mM NaCl and centrifugation at 30 Krpm at 4°C during 45 min in a 65 Ty rotor. This soluble fraction was precipitated by addition of 60% of ammonium sulfate and centrifugation at 40 Krpm at 4°C for 60 min. The precipitated fraction was then resuspended in 1 ml of 20 mM Tris-HCl pH 7.5, 500 mM KCl) and dialysed against the same buffer to eliminate the ammonium sulfate. The dialysed fraction was loaded in a 5ml fast-flow chelating sepharose (Pharmacia) activated with Ni²⁺ and equilibrated with the dialysis buffer in which the HisKis-Kid complex was retained. A gradient of 0 to 6 M of guanidinium cloride (GnCl) in 20 mM Tris-HCl pH 7.5 was applied to the column and denaturation of the HisKis-Kid complex bound to the column led to retention of HisKid and elution of Kid at 5.5 M of the chaotropic agent. Denatured Kid can be stored at -80°C until necessary. For renaturation, Kid was diluted to 5 pmol/µl in 6 M GnCl, 150 mM CIK, 100 mM phosphate buffer pH 6.5, 20 mM β-mercaptoethanol, 0.2 mM EDTA and 1.2 % CHAPS and dialysed 5 times during 6 hours at 4°C against 200 ml (per 6 ml of protein) of 100 mM phosphate buffer pH 6.5, 150 mM KC1, 10 mM β-mercaptoethanol, 0.1 mM DTT and 10 % ethyleneglycol. The soluble and refolded protein was separated from the insoluble (denatured) one by centrifuging the mix at 40 Krpm for 60 min at 4°C in a 65 Ty rotor. The supernatant was concentrated in centricon tubes (cut off 3 K) and aliquoted after determining purity and concentration of the protein by coomassie staining on a SDS-PAGE gel and spectrophotometric analysis, respectively, and stored at -80°C.

### Embryo microinjections

MBPKis and Kid proteins were dialysed against buffer 20 mM Tri-HCl pH 8.0, 50 mM Kcl and 2 µl of MBPKid (160 ng/µl) and 2 µl of MBPKis (720 ng/µl) were mixed with each other or with 2 µl of dialysis buffer and incubated on ice for 10 min. 50 nl of each mix (buffer, Kis, Kid and Kis/Kid) were microinjected into dejellied two cell embryos of Xenopus laevis at the animal pole of one of their cells. Microinjected and non-injected embryos were then incubated in 4% of ficoll 400 in MBS buffer at 18°C and allow to progress through embryonic development until stage 8-9 (blastula) was reached in the case of the non-injected controls (7-8 hours). Embryos were then photographed and the effect of microinjections analysed afterwards.

### HeLa cells

### Plasmids (pNATHAs)

Oligonucleotides EcoRIKis (5' CGGAATTCATGCATACTACCACCCGACTG3' - SEQ ID NO. 10) or EcoRINLSKis (5' CGGAATTCATGGACAAGGTTCCTAAGAAGAAGAGGAAGGTTAGCAGCATGCATACCACC CGACTGAAG3' - SEQ ID NO. 11) and KisXbal (5'CTCTAGATCAGATTTCCTCCTGACC3' - SEQ ID NO. 12) were used to amplify kis by PCR using a mini-R1 plasmid as template. The amplified product was digested with EcoRI and XbaI and cloned in pTRE plasmid (Clontech) between EcoRI and Xbal sites to obtain the pTREKis and pTRENLSKis plasmids, respectively. On the other hand, oligonucleotides XholKid (5'CCGCTCGAGATGGAAAGAGGGGAAATCT3' - SEQ ID NO. 13) and KidEcoRI (SEQ ID NO. 5) were used to amplify kid by PCR using a mini-R1 plasmid as template, and EcoRIKid (5'CGGAATTCATGGAAAGAGGGGAAATCT3' - SEQ ID NO. 14) and KidNLSXbal (5'GCTCTAGATCAAACCTTCCTCTTCTTCTTAGGAGGCCTGCTGCTAGTCAGAATAGTGGA CAGGCG3' - SEQ ID NO. 15) were used with the same purpose to obtain an NLSKid gene by PCR using a mini-R1 plasmid as template. These two PCR products were digested with XhoI and EcoRI or EcoR1 and XbaI, respectively, and cloned between these sites in the plasmid pClneo (Promega) to obtain the plasmids pClneoKid and pClneoKidNLS. These kid+ plasmids were amplified in a bacterial strain that overproduces Kis at the same time to abolish selection of inactivating mutants during the cloning process. Fragment BsTXI-Smal was deleted from pClneoKid and pClneoKidNLS to eliminate the neomycin resistance gene. The resultant plasmids (pCIKid and pCIKidNLS) were digested with BgIII and BamHI and treated with Klenow, and the fragment containing the kid or kidNLS genes were purified and cloned in the pTRE and pTREKis vectors digested with HindIII and treated with Klenow. For each of these constructs both orientations were selected, and plasmids pNATHA1 (kis+), pNATHA2 (kis+/kid+), pNATHA 4 (NLSkis+) and pNATHA 8 (NLSkis+/kidNLS+) were obtained both in kis-kid tail-to-tail (pNATHAi) and tail-to-head (pNATHAd) orientations.

### Selection of stable transfectants

5 µg of each pNATHA was mixed with 0.5 µg of pTKHyg plasmid and HeLa Tet-Off cell line (Clontech) was transfected with these mixtures by the Lipofectamine method (Gibco). Stable transfectants were selected in DMEM medium suplemented with glutamax and 10% of tetracyclin approved fetal bovine serum (Clontech) and in the presence of 200 µg/ml of neomycin (Sigma) and 200 µg/ml of hygromycin (Clontech) (non-toxic medium; NTM).

### In vivo assays

### Cell growth and death rate determination

HeLa Tet Off cells stably transfected with pNATHAli+ and pNATHA2i+ were grown in NTM until they reached aproximately 80% of confluency. They were trypsinised and 5x10 pNATHAi1+ and 2x10⁴ pNATHAi2+ stably transfected cells were transfered to 4 wells of a six multiwell plate and grown for 24 hours in NTM. After that, one of the wells per sample was trypsinised and these cells pelleted and stained with trypan blue. Total and trypan blue stained (dead) cells per well were counted with a cytometer. Then, 0.1 µg/ml of Doxycycline (Sigma) was added to the rest of wells and cells were allowed to grow in this toxic medium (TM) for 2, 5 and 10 days, changing it each 4 days when necessary but retaining the floating (dead and mitotic) cells each time that fresh TM was added. Trypsinisation, trypan blue staining and counting of cells was repeated for each sample to determine the total and dead number of cells per sample.

### Annexin V staining

HeLa Tet Off cells stably transfected with pNATHA1+ and pNATHA2+ were grown in NTM until they reached aproximately 80% of confluency. They were trypsinised and 10⁴ pNATHAi1+ and 5X10⁴ pNATHAi2+ stably transfected cells were transfered to four dishes (two per sample) of 5 cm of diameter in which four polylysine coated coverslips were placed. Cells were allowed to settle down for 24 hours and then 0.1 µg/ml of doxycyclin was added to one of the dishes per sample. Coverslips were taken out from the dishes before (day 0) and 2, 5 and 10 days after addition of doxycyclin to one of them. Fresh medium was added each 4 days if necessary. Samples growing on these coverslips were stained with FITC-Annexin V (Clontech) as suggested by the manufacturer, before fixing them, and DNA was stained with propidium iodide and Hoeschts 33258. Analysis and counting of annexin V positive cells was done by confocal microscopy and total and apoptotic number of cells was determined.

## Claims

1. A composition comprising:
(i) the ParD kid toxin and ParD kis antitoxin, or
(ii) nucleic acid encoding the ParD kid toxin and ParD kis antitoxin,
for use in a therapeutic method of inhibiting cell proliferation and/or cell cycle progression carried out on a human or animal body, the method comprising providing within eukaryotic cells in the human or animal body the toxin and antitoxin, under appropriate control for selective cell cycle inhibition and/or killing of target cells.

2. A composition according to claim 1 wherein said toxin is to be provided within said cells by means of nucleic acid encoding said toxin under control of appropriate control elements for expression.

3. A composition according to any one of the preceding claims, for use in the method comprising providing to said cells said toxin and said antitoxin and controlling activity of said antitoxin on said toxin to control activity of said toxin on said cells.

4. A composition according to claim 3 wherein said antitoxin is to be provided within said cells by means of nucleic acid encoding said antitoxin under control of appropriate control elements for expression.

5. A composition according to claim 3 or claim 4 wherein the selectivite cell cycle inhibition and/or killing is effected by a combination of (i) up-regulation of toxin production in target cells and (ii) down-regulation of toxin production in non-target cells and/or neutralisation of toxin activity in non-target cells.

6. A composition according to claim 5 wherein neutralisation of toxin activity in non-target cells is effected by upregulation of antitoxin production in non-target cells.

7. A composition according to any one of claims 3 to 6 wherein said target cells are tumour cells.

8. Use of a composition comprising:
(i) the ParD kid toxin and ParD kis antitoxin, or
(ii) nucleic acid encoding the ParD kid toxin and ParD kis antitoxin,
in the manufacture of a medicament composition according to the composition of any one of claims 1 to 7 for treatment of tumours, cancer, psoriasis, arteriosclerosis or other hyper-proliferative disorder.

9. A method of inhibiting cell proliferation and/or cell cycle progression, the method comprising providing within eukaryotic cells the ParD kid toxin and ParD kis antitoxin under appropriate control for selective cell cycle inhibition and/or killing of target cells, wherein the cells are *in vitro* and/or are plant cells.

10. A method according to claim 9 wherein said toxin is provided within said cells by means of nucleic acid encoding said toxin under control of appropriate control elements for expression.

11. A method of inhibiting cell proliferation and/or cell cycle progression, the method comprising providing within eukaryotic cells the ParD kid toxin and ParD kis antitoxin, wherein the cells are *in vitro* and/or are plant cells.

12. A method according to any one of claims 9 to 11 comprising providing to said cells the ParD kid toxin and ParD kis antitoxin, and controlling activity of said antitoxin on said toxin to control activity of said toxin on said cells.

13. A method according to claim 12 wherein said antitoxin is provided within said cells by means of nucleic acid encoding said antitoxin under control of appropriate control elements for expression.

14. A method according to claim 12 or claim 13 wherein selectivity for expression of said toxin within target cells is effected by a combination of (i) up-regulation of toxin production in target cells and (ii) down-regulation of toxin production in non-target cells and/or neutralisation of toxin activity in non-target cells.

15. A method according to claim 14 wherein neutralisation of toxin activity in non-target cells is effected by upregulation of antitoxin production in non-target cells.

16. A method according to any one of claims 12 to 14 wherein said target cells are tumour cells.

## Patentansprüche

1. Zusammensetzung, umfassend
(i) ParD-kid-Toxin und ParD-kis-Antitoxin oder
(ii) für das ParD-kid-Toxin und parD-kis-Antitoxin kodierende Nucleinsäure
zur Verwendung in einem therapeutischen Verfahren zur Hemmung der Zellproliferation und/oder Zellzyklusprogression, das auf einem menschlichen oder tierischen Körper durchgeführt wird, wobei das Verfahren die Bereitstellung des Toxins und Antitoxins in eukaryotischen Zellen im menschlichen oder tierischen Körper unter geeigneter Kontrolle zur selektiven Zellzyklusinhibition und/oder Tötung der Targetzellen umfasst.

2. Zusammensetzung nach Anspruch 1, worin das Toxin in den Zellen durch Nucleinsäure, die für das Toxin kodiert, unter der Kontrolle geeigneter Expressionskontrollelemente bereitgestellt werden soll.

3. Zusammensetzung nach einem der vorangegangenen Ansprüche zur Verwendung im Verfahren, das die Bereitstellung des Toxins und des Antitoxins für diese Zellen sowie die Kontrolle der Aktivität des Antitoxins auf dem Toxin umfasst, um die Aktivität des Toxins auf diesen Zellen zu kontrollieren.

4. Zusammensetzung nach Anspruch 3, worin das Antitoxin in diesen Zellen durch Nucleinsäure, die für das Antitoxin kodiert, unter der Kontrolle geeigneter Expressionskontrollelemente bereitgestellt werden soll.

5. Zusammensetzung nach Anspruch 3 oder 4, worin die selektive Zellzyklusinhibition und/oder Tötung durch eine Kombination von (i) Hinaufregulierung von Toxinproduktion in Targetzellen und (ii) Herabregulierung von Toxinproduktion in Nicht-Targetzellen und/oder Neutralisierung von Toxinaktivität in Nicht-Targetzellen ausgeführt wird.

6. Zusammensetzung nach Anspruch 5, worin die Neutralisierung von Toxinaktivität in Nicht-Targetzellen durch Hinaufregulierung von Antitoxinproduktion in Nicht-Targetzellen ausgeführt wird.

7. Zusammensetzung nach einem der Ansprüche 3 bis 6, worin die Targetzellen Tumorzellen sind.

8. Verwendung einer Zusammensetzung, die Folgendes umfasst:
(i) das ParD-kid-Toxin und ParD-kis-Antitoxin oder
(ii) Nucleinsäure, die für das ParD-kid-Toxin und ParD-kis-Antitoxin kodiert,
zur Herstellung einer Arzneimittelzusammensetzung gemäß der Zusammensetzung aus einem der Ansprüche 1 bis 7 zur Behandlung von Tumoren, Krebs, Psoriasis, Arteriosklerose oder anderen hyperproliferativen Erkrankungen.

9. Verfahren zur Hemmung der Zellproliferation und/oder Zellzyklusprogression, wobei das Verfahren die Bereitstellung des ParD-kid-Toxins und ParD-kis-Antitoxins in eukaryotischen Zellen unter einer geeigneten Kontrolle zur selektiven Zellzyklusinhibition und/oder Tötung von Targetzellen umfasst, worin die Zellen in vitro sind und/oder Pflanzenzellen sind.

10. Verfahren nach Anspruch 9, worin das Toxin in diesen Zellen durch Nucleinsäure, die für das Toxin kodiert, unter der Kontrolle von geeigneten Expressionskontrollelementen bereitgestellt wird.

11. Verfahren zur Hemmung von Zellproliferation und/oder Zellzyklusprogression, wobei das Verfahren die Bereitstellung des ParD-kid-Toxins und ParD-kis-Antitoxins in eukaryotischen Zellen umfasst, worin die Zellen in vitro sind und/oder Pflanzenzellen sind.

12. Verfahren nach einem der Ansprüche 9 bis 11, das die Bereitstellung des ParD-kid-Toxins und ParD-kis-Antitoxins für die Zellen und die Kontrolle der Aktivität des Antitoxins auf dem Toxin zur Kontrolle der Aktivität des Toxins auf den Zellen umfasst.

13. Verfahren nach Anspruch 12, worin das Antitoxin in den Zellen durch Nucleinsäure, die für das Antitoxin kodiert, unter der Kontrolle geeigneter Expressionskontrollelemente bereitgestellt wird.

14. Verfahren nach Anspruch 12 oder Anspruch 13, worin die Selektivität für die Expression des Toxins in Targetzellen durch eine Kombination von (i) Hinaufregulierung von Toxinproduktion in Targetzellen und (ii) Herabregulierung von Toxinproduktion in Nicht-Targetzellen und/oder Neutralisierung von Toxinaktivität in Nicht-Targetzellen erreicht wird.

15. Verfahren nach Anspruch 14, worin die Neutralisierung von Toxinaktivität in Nicht-Targetzellen durch eine Hinaufregulierung von Antitoxinproduktion in Nicht-Targetzellen erreicht wird.

16. Verfahren nach einem der Ansprüche 12 bis 14, worin die Targetzellen Tumorzellen sind.

## Revendications

1. Composition comprenant:
(i) la toxine ParD kid et l'anti-toxine Pard kis, ou
(ii) un acide nucléique codant la toxine ParD kid et l'anti-toxine ParD kis,
à utiliser dans un procédé thérapeutique pour inhiber la prolifération cellulaire et/ou la progression du cycle cellulaire, appliqué à un corps humain ou animal, le procédé comprenant les étapes consistant à fournir, dans les cellules eucaryotes du corps humain ou animal, la toxine et l'anti-toxine, sous contrôle approprié, pour une inhibition sélective du cycle cellulaire et/ou pour tuer des cellules cibles.

2. Composition selon la revendication 1 dans laquelle ladite toxine doit être fournie dans lesdites cellules au moyen d'un acide nucléique codant ladite toxine sous le contrôle d'éléments de contrôle de l'expression appropriés.

3. Composition selon l'une quelconque des revendications précédentes, à utiliser dans le procédé comprenant les étapes consistant à fournir aux dites cellules ladite toxine et ladite anti-toxine et à contrôler l'activité de ladite anti-toxine sur ladite toxine pour contrôler l'activité de ladite toxine sur lesdites cellules.

4. Composition selon la revendication 3 dans laquelle ladite anti-toxine doit être fournie dans lesdites cellules au moyen d'un acide nucléique codant ladite anti-toxine sous le contrôle d'éléments de contrôle de l'expression appropriés.

5. Composition selon la revendication 3 ou la revendication 4 dans laquelle l'inhibition sélective du cycle cellulaire et/ou l'élimination sont réalisées par une combinaisons (i) d'une régulation à la hausse de la production de toxine dans les cellules cibles et (ii) une régulation à la baisse de la production de toxine dans les cellules non cibles et/ou une neutralisation de l'activité de la toxine dans les cellules non cibles.

6. Composition selon la revendication 5 dans laquelle la neutralisation de l'activité de la toxine dans les cellules non cibles est réalisée par une régulation à la hausse de la production d'anti-toxine dans les cellules non cibles.

7. Composition selon l'une quelconque des revendications 3 à 6 dans laquelle lesdites cellules cibles sont des cellules tumorales.

8. Utilisation d'une composition comprenant:
(i) la toxine ParD kid et l'anti-toxine Pard kis, ou
(ii) un acide nucléique codant la toxine ParD kid et
l'anti-toxine ParD kis,
pour la fabrication d'une composition médicamenteuse selon la composition de l'une quelconque des revendications 1 à 7 pour le traitement de tumeurs, de cancers, du psoriasis, de l'artériosclérose ou d'autres maladies d'hyperprolifération.

9. Procédé pour inhiber la prolifération cellulaire et/ou la progression du cycle cellulaire, le procédé comprenant les étapes consistant à fournir, dans des cellules eucaryotes, la toxine ParD kid et l'anti-toxine ParD kis sous contrôle approprié, pour une inhibition sélective du cycle cellulaire et/ou pour tuer des cellules cibles, les cellules cibles étant des cellules *in vitro* et/ou étant des cellules végétales.

10. Procédé selon la revendication 9 dans lequel ladite toxine est fournie dans lesdites cellules au moyen d'un acide nucléique codant ladite toxine sous le contrôle d'éléments de contrôle de l'expression appropriés.

11. Procédé pour inhiber la prolifération cellulaire et/ou la progression du cycle cellulaire, le procédé comprenant les étapes consistant à fournir, dans des cellules eucaryotes, la toxine ParD kid et l'anti-toxine ParD kis, les cellules étant des cellules *in vitro* et/ou des cellules végétales.

12. Procédé selon l'une quelconque des revendications 9 à 11 comprenant les étapes consistant à fournir aux dites cellules la toxine ParD kid et l'anti-toxine ParD kis et à contrôler l'activité de ladite anti-toxine sur ladite toxine pour contrôler l'activité de ladite toxine sur lesdites cellules.

13. Procédé selon la revendication 12 dans lequel ladite anti-toxine est fournie dans lesdites cellules au moyen d'un acide nucléique codant ladite anti-toxine sous le contrôle d'éléments de contrôle de l'expression appropriés.

14. Procédé selon la revendication 12 ou la revendication 13 dans lequel la sélectivité pour l'expression de ladite toxine dans les cellules cibles est obtenue en combinant (i) une régulation à la hausse de la production de toxine dans les cellules cibles et (ii) une régulation à la baisse de la production de toxine dans les cellules non cibles et/ou une neutralisation de l'activité de la toxine dans les cellules non cibles.

15. Procédé selon la revendication 14 dans lequel la neutralisation de l'activité de la toxine dans les cellules non cibles est obtenue par régulation à la hausse de la production d'anti-toxine dans les cellules non cibles.

16. Procédé selon l'une quelconque des revendications 12 à 14 dans lequel lesdites cellules cibles sont des cellules tumorales.
